# EUROPEAN PATENT SPECIFICATION

(11) **EP 4 563 110 B1**
(45) Date of publication and mention of the grant of the patent: **02.09.2026**
(21) Application number: 24210800.9
(22) Date of filing: 05.11.2024
(51) Int. Cl.: A61B 18/18

(54) **COOLANT STATUS MONITORING FOR MICROWAVE ABLATION PROBE**
KÜHLMITTELZUSTANDSÜBERWACHUNG FÜR MIKROWELLENABLATIONSSONDE
SURVEILLANCE DE L'ÉTAT D'UN LIQUIDE DE REFROIDISSEMENT POUR SONDE D'ABLATION PAR MICRO-ONDES

(30) Priority: 29.11.2023 US 202318523762
(43) Date of publication of application: 04.06.2025
(73) Proprietor: Varian Medical Systems, Inc., Palo Alto, CA 94304 (US)
(72) Inventor: FALLAHI, Hojjatollah, Minneapolis, 55401 (US); WEINZIERL, David, Andover, 55304 (US); OCHOCO, Jomar, St. Paul, 55104 (US)
(74) Representative: Mathisen & Macara LLP

(56) References cited:
- US-A1- 2005 222 556
- US-A1- 2016 008 069
- US-A1- 2020 155 225
- US-A1- 2020 391 051

## Description

### BACKGROUND

Embodiments of the disclosure generally relate to electromagnetic radiation (EMR) therapy and more particularly to monitoring the cooling of a microwave ablation probe used to apply electromagnetic energy to a treatment site to heat tissue needing treatment at the treatment site.

The use of electromagnetic (EM) energy to heat tissue for the treatment of disease is known. In using microwave energy for tissue heating, an applicator or probe having a microwave radiating antenna is positioned with respect to the tissue to be treated (heated) so that microwave energy radiated from the antenna penetrates and heats/ablates the tissue. Many microwave probes are known in the art. Death, or necrosis, of living tissue cells occurs at temperatures elevated above a normal cell temperature for a sufficient period of time. The sufficient period of time is generally dependent upon the temperature to which the cells are heated. Above a threshold temperature of about 41.5° C, substantial thermal damage occurs in most malignant cells. At temperatures above about 45° C, thermal damage occurs to most normal cells. During treatment, it is desirable to produce an elevated temperature within the target tissue for a time period sufficient to cause the desired cell damage, while keeping nearby healthy tissue at a safe, lower temperature. For this reason, when treatment involving tissue heating is used, it is important to assure both (i) adequate tumor/diseased tissue heating throughout the tumor/diseased to the tumor/diseased tissue margin and (ii) reduced temperatures in the normal/undiseased tissue surrounding the tumor/diseased tissue.

Heating therapy is sometimes combined with other treatments, such as surgery, ionizing radiation, and chemotherapy. For example, when heating is combined with radiation, it is desirable to maintain the temperature within the diseased tissue within the range of about 42° C to 45° C. Higher temperatures are usually undesirable when a combined treatment modality is used because higher temperatures can lead to microvessal collapse causing resistance to other therapies such as, radiation therapy and chemotherapy, and decrease the amount of systemic chemotherapy from reaching the tumor as a result of vascular damage. Lower temperatures are undesirable because these temperatures can fail to provide adequate heating and thereby, reduce the therapeutic effect of the treatment. Therefore, it is important to control the temperature within the desired range for multi-modality treatments and not allow heating of the tissue in the tumor/diseased tissue or surrounding the tumor to temperatures above about 45° C because heating the tissue to these temperatures may damage the tissue, compromising the effectiveness of other treatments. Treatment within this controlled temperature range is usually referred to as hyperthermia.

Forms of thermal therapy that kill the tissue with heating alone are generally referred to as coagulation or ablation. To adequately eradicate a cancerous tumor/diseased tissue with only the application of heat, it is necessary to ensure sufficient heating is accomplished throughout the entire tumor/tissue. In cases of a malignant tumor, if viable tumor cells are left behind, the tumor can rapidly grow back leaving the patient with the original problem. In what is generally referred to as microwave coagulation or microwave ablation, the diseased tissue is heated to at least about 55° C, and typically to above about 60° C, for exposure times sufficient to kill the cells, typically, for greater than about one minute. With microwave coagulation and ablation treatments, there is a volume reduction of temperature that ranges from the high temperature in the treated tissue to the normal (healthy) tissue temperature of 37° C outside the treated tissue.

For proper treatment of targeted cancerous tumor volumes or other tissue volumes to be treated, it becomes very important to properly deliver the correct, targeted thermal distribution over a sufficient time period to eradicate the tumor tissue while minimizing damage to critical surrounding normal tissue. Fortunately, there are tumor locations that reside in normal tissue that can be destroyed by the heating in limited areas without affecting the health of the patient, such as liver tissue. In such situations the coagulation can be applied in an aggressive way to include a margin of safety in destruction of limited surrounding normal tissues to assure that all of the cancerous tumor/diseased tissue is destroyed.

The process of heating very rapidly to high temperatures that is common in coagulation and ablation treatments can utilize a rather short exposure time. In doing so, the resulting temperature distribution becomes primarily a result of the power absorption distribution within the tissue. However, if such treatments continue for multiple minutes, the blood flow and thermal conduction of the tumor/diseased tissue and surrounding tissues will modify the temperature distribution to result in a less predictable heat distribution because the changes occurring in blood flow in such a heated region, may not be predictable. Therefore, it is important to optimize the uniformity of the tissue heating power that is absorbed to lead to a more predictable temperature distribution that better corresponds with the treatment prescription. Therefore, pretreatment planning practices prior to and possibly during treatment for calculating the power and temperature distribution resulting from the parameters of power and relative phase of the power applied to the tissue can be important for not only coagulation and ablation, but also hyperthermia. As higher temperatures are used during treatment, it may increase patient discomfort and pain, so it can be helpful to avoid excessive temperatures to reduce the need of patient sedation.

Minimally invasive microwave energy probes can be inserted into living body tissue to place the source of heating into or adjacent to a diseased tissue area. Minimally Invasive probes help to overcome some difficulties that surface applicators experience when the target tissue region is located below the skin (e.g., the prostrate). Minimally invasive probes must be properly placed to localize the heating to the vicinity of the desired treatment area. Even when properly placed, however, it has been difficult to ensure that adequate heat is developed in the diseased tissue without overheating surrounding healthy tissue. Further, with probes operating at higher power levels to produce the needed higher temperatures for coagulation and ablation, there is a tendency for the microwave transmission line (coaxial cable) in the portion of the probe leading from outside the body to the location of the radiating antenna in the probe to heat to undesirably high temperatures, which can cause thermal damage to the normal tissue through which the probe passes to reach the diseased tissue to be treated. In addition, to keep the probe form factor minimally invasive, small profile coaxial cables are used which are not capable of transferring microwave energy at high levels without cooling the cables in the probe. Therefore, various ways of cooling the probe have been used with prior devices.

Cooling an ablation probe can enhance the overall heating pattern of the antenna, prevent damage to the antenna and prevent harm to the clinician or patient. Because of high temperatures that can be achieved by microwave ablation probes and susceptibility of cells to these temperatures, a known heating pattern and precise temperature control is needed to lead to more predictable temperature distribution to eradicate the tumor cells while minimizing the damage to surrounding normal tissue.

Fluid cooled microwave probes can be used in ablation procedures, see for instance US2016/008069 A1 or US2020/391051 A1. During operation of a microwave ablation device, if proper cooling is not maintained, e.g., flow of coolant within the probe is interrupted, the microwave ablation device can overheat and cause damage to the coaxial cable or radiating antenna in the device which in turn leads to high reflected power and irreversible failure of the device. A coolant fluid having gas bubbles can be circulated from a cooling system internal to a microwave ablation probe. When a bubble filled with even a small volume of air or multiple air bubbles in close proximity to each other are introduced into an ablation probe, e.g., when the ablation probe is operating at a high power level, the ablation probe can be susceptible to rapid failure due to overheating or arcing conditions. The time to failure is dependent on the power delivered to the antenna and degree to which coolant flow is reduced and/or the duration of the interruption.

While many microwave ablation probes are known in the art for applying microwave energy to tissue to provide heating to the tissue, there is a need for better thermal control in order to perform more efficient, consistent, and safe ablations.

### SUMMARY

The invention is defined in the appended set of claims. Advantageous embodiments of the invention are explained hereinafter. Embodiments are directed to a microwave ablation probe that includes an elongate shaft; a coupling attached to the elongate shaft and a coolant reservoir; and a flexible circuit including a thermal sensor and attached to the elongate shaft. The microwave alation probe may comprise an array of thermal sensors. The multiple thermal sensors can be positioned at various locations on the probe. The thermal sensor(s) may be a thermocouple, thermistor, etc.). Suitably the thermal sensors are on the same substrate. The sensors may be located on the substrate at predetermined geometric locations. An array of thermal sensors may include a plurality of sensors on the body of the probe as well as an inflow and outflow sensor. The inflow and outflow sensors can be adhered to the coupling.

The flexible circuit may include a flexible substrate. The flexible substrate may have metalized traces routed to define an electrical circuit. A thermal sensor can be located on cables/tubes routed between the microwave ablation probe and an associated control console. The flexible circuit may include a plurality of legs, such as three legs. In an aspect, the flexible circuit is attached to the coupling and includes a thermal sensor, such as, a thermocouple at the coupler. The flexible circuit may be adhered to the probe shaft. A heat shrink may be slid over the probe shaft.

A connector is implemented on the same PCB as the sensor. This omits the soldering step on conventional thermocouple wires to connectors or printed circuit boards. Suitably the thermal sensors can be routed to terminal pads for interface to a connector. The connector and or terminal pads may be integrated into the flexible substrate.

Multiple sensors are implemented on the same PCB and no separate wires needed for each sensor as in conventional thermocouple wires. This facilitates assembly and control in manufacturing.

In an embodiment, a microwave ablation probe system includes a coolant delivery system to deliver coolant to a microwave ablation probe; a microwave generator to deliver microwave energy to the microwave ablation probe; and a computing system in communication with a thermal sensor on the microwave ablation probe to collect temperature data over time from the thermal sensor, wherein the computing system is configured to calculate a slope of the temperature data over time to determine if the coolant delivery system is cooling the microwave ablation probe. The microwave ablation system can include a microwave ablation controller, an ablation computing system, a coolant reservoir, a flexible cable and the microwave ablation probe. The flexible cable may couple the ablation controller to the microwave ablation probe. The flexible cable can include an inflow line an outflow line, sensor wiring and microwave wiring. The flexible cable can include connectors suitable to connect to the ablation controller and the microwave ablation probe.

The microwave ablation probe system can further include a flexible circuit that includes the thermal sensor.

In an aspect, the flexible circuit is attached to the microwave ablation probe.

In an aspect, the flexible circuit includes a plurality of thermal sensors.

In an aspect, the coolant delivery system includes a conduit through the microwave ablation probe.

In an aspect, the microwave ablation probe includes a coupling to which the conduit is attached.

Another embodiment provides, a method to measure coolant status of a microwave ablation probe, the method includes activating a coolant delivery system to deliver coolant to the microwave ablation probe; activating microwave energy to the microwave ablation probe; measuring temperature of the microwave ablation probe over time; calculating a slope of the temperature over time; and comparing the slope to a predetermined value.

In an aspect, the method further comprises if the slope is at or greater than the predetermined value, troubleshooting the coolant delivery system to verify that coolant is being delivered to the microwave ablation probe, and if the slope is less than the predetermined value, determining if the slope is negative after being positive.

In an aspect, the method further comprises if the slope is not negative after being positive, troubleshooting the coolant delivery system to verify that coolant is being delivered to the microwave ablation probe, and if the slope is negative after being positive, using the microwave ablation probe.

As a result, such microwave ablation probes and methods to measure coolant status of a microwave ablation probe permit greater temperature control of ablation zones with increased safety to the patient and care providers. Additional advantages include a lower cost configuration of providing multiple thermocouples in flexible circuitry compared to a single thermocouple circuit built using wires; omission of a soldering step that connects two different metals in thermocouple wires in order to create the sensing tip of the thermocouple; and increased mechanical integrity as a flexible circuit is not fragile like discrete thin thermocouple wires. These advantages lead to higher yield in fabricating a microwave ablation probe with integrated thermal sensing, less labor, and more accurate placement of thermal sensors on the microwave ablation probe.

The description, objects and advantages of embodiments of the present invention will become apparent from the detailed description to follow, together with the accompanying drawings.

### BRIEF DESCRIPTION OF THE DRAWINGS

The above-mentioned aspects, as well as other features, aspects and advantages of the present technology will now be described in connection with various embodiments, with reference to the accompanying drawings. The illustrated embodiments, however, are merely examples and are not intended to be limiting. Throughout the drawings, similar symbols typically identify similar components, unless context dictates otherwise. Note that the relative dimensions of the following FIGS. may not be drawn to scale.
FIG. 1 is a side view of an exemplary microwave ablation probe.
FIG. 2 is a side view of a microwave ablation probe according to an embodiment of the current disclosure.
FIG. 3 is top view of a flexible circuit.
FIG. 4 is a side view of a microwave ablation probe including a flexible circuit.
FIG. 5 is a side view of a microwave ablation probe used for experimentation.
FIG. 6 is a block diagram of a microwave ablation probe system according to an embodiment of the current disclosure.
FIG. 7 is a graph of time versus thermocouple temperature with no coolant applied.
FIG. 8 is a graph of slope of thermocouple temperatures from FIG. 6.
FIG. 9 is a graph of time versus thermocouple temperature evaluating coolant priming.
FIG. 10 is a flow chart of a method of monitoring a coolant status of a microwave ablation probe according to an embodiment of the present disclosure.

### DETAILED DESCRIPTION

It is to be understood that the embodiments of the invention described herein are not limited to particular variations set forth herein as various changes or modifications may be made to the embodiments of the invention described hereinafter. As will be apparent to those of skill in the art upon reading this disclosure, each of the individual embodiments described and illustrated herein has discrete components and features that may be readily separated from or combined with the features of any of the other several embodiments without departing from the scope of the embodiments of the present invention. In addition, many modifications may be made to adapt a particular situation, material, composition of matter, process, process act(s) or step(s) to the objective(s), or scope of the embodiments of the present invention. All such modifications are intended to be within the scope of the claims made herein.

Moreover, while methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It will be understood that when an element is referred to as being "connected" or "coupled" to another element, it can be directly connected or coupled to the other element or intervening elements may be present. In contrast, if an element is referred to as being "directly connected" or "directly coupled" to another element, there are no intervening elements present.

It will also be understood that, although the terms first, second, etc. may be used herein to describe various elements, these elements should not be limited by these terms. These terms are only used to distinguish one element from another. Thus, a first element could be termed a second element without departing from the teachings of the present invention.

Language of degree used herein, such as the terms "approximately," "about," "generally," and "substantially," represent a value, amount, or characteristic close to the stated value, amount, or characteristic that still performs a desired function or achieves a desired result. For example, the terms "approximately," "about," "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. Additionally, numeric ranges are inclusive of the numbers defining the range, and any individual value provided herein can serve as an endpoint for a range that includes other individual values provided herein. For example, a set of values such as 1, 2, 3, 8, 9, and 10 is also a disclosure of a range of numbers from 1-10, from 1-8, from 3-9, and so forth.

While a number of embodiments and variations thereof have been described in detail, other modifications and methods of using the same will be apparent to those of skill in the art. Accordingly, it should be understood that various applications, modifications, materials, and substitutions can be made without departing from the unique and inventive disclosure herein or the scope of the claims.

Thermal ablation can be used to treat many conditions and diseases including, and not limited to, cancerous tissue. These conditions and diseases can be treated in many organs of the human body including, and not limited to, the liver, lungs, kidneys, prostate, bladder, ovaries, cervix, uterus, endometrium, breasts, brain, stomach, colon and skin. In treating certain conditions, it is imperative to ensure ablation of the diseased tissue while not damaging healthy tissue adjacent to the ablation site. Accordingly, embodiments of the present disclosure are directed to microwave applicator probes that have a more predictable and controlled ablation zone.

In fluid-cooled microwave ablation probes it is important to monitor the status of the fluid in terms of presence of fluid in the probe, detection of sufficient flow, occurrence of occlusion in the fluid lines, and fluid temperature for effective treatment outcome. In addition, probe shaft temperature must stay under the safety limit in non-active portions of the probe (i.e., outside the ablation zone with heat created by microwaves). The maximum temperature ranges from 43° to 48° C depending on the heating duration. For example, the shaft temperature could be at 43° C for about 11 minutes without damaging tissue. While, on the other hand, the shaft temperature can damage tissue at 48° C at about 1.5 minutes.

In addition, in current clinical practice, X-ray Computed Tomography (CT) and ultrasound (US) imaging are the most widely used modalities for guiding probe placement and are also used to assess technical success of the procedure. One of the current challenges with microwave ablation is a lack of established techniques for intra-procedural monitoring to determine treatment endpoint, and this remains an area of active investigation.

In some fluid-cooled microwave ablation probes, fluid can circulate in a closed looped circuit between a reservoir and the probe. The probe system can include a spike that is connected to a saline bag as a coolant reservoir. The spike has inflow and outflow paths integrated in it. If there are no sensors, the user starts fluid flow and observes the flow output on the spike. In the beginning, bubbles appear in the saline bag from the outflow path in the spike. When bubbles are no longer observed, fluid is assumed to be present within the probe and all the tubing between the spike and the probe.

Disclosed embodiments utilize an array of thermal sensors (i.e., thermocouple, thermistor, etc.) to monitor the temperature at different points on the microwave ablation probe assembly including points on the probe shaft outside and inside the ablation zone, surface of the RF cable, and in the coolant inflow and outflow paths. In addition, thermal sensor locations are optimized to provide feedback to the user on the progress of the ablation zone growth.

These multiple thermal sensors can be positioned at various locations on the probe to provide temperature information that can be used to provide feedback of operating characteristics of the probe and related systems, such as the cooling system. The temperature information can be used, for example, to determine when the probe and/or cooling system has reached predetermined operating parameters during a start-up phase of a treatment. In other examples, the temperature information can be used to determine a size and/or shape of the ablation zone. In other examples, the temperature information can be used to determine whether an occlusion, blockage, or other failure has occurred in the cooling system. In yet other examples, the temperature information can be used to adjust or modify operating parameters during an ablation treatment. In still other examples, the temperature information can be used to determine when an ablation treatment is completed.

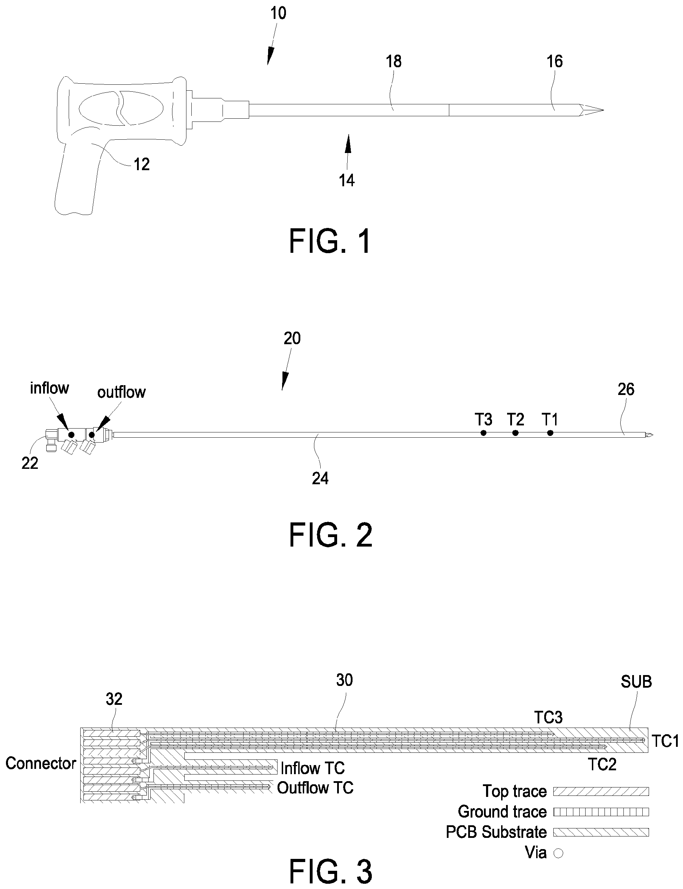

FIG. 1 is an example of a microwave ablation probe 10. The microwave ablation probe 10 can includes a handle 12 from which a substantially rigid elongate applicator body 14 extends with an insertion tip 16 forming the insertion end portion of the probe for insertion into a region of the target tissue to be ablated. The handle 12 can be define as a pistol grip shown, another suitable shape, or be omitted completely. The substantially rigid elongate applicator body 14 can include an outer sleeve 18 extending from the handle 12.

An embodiment of a microwave ablation probe for microwave coagulation and ablation treatment of diseased tissue within living body tissue according to the present disclosure is illustrated in FIG. 2. As shown, the microwave ablation probe 20 can include a coupling 22 and a substantially rigid elongate probe body 24 that extends to an insertion tip 26 forming the insertion end portion of the probe for insertion into a region of the target tissue to be ablated. Although not shown, a handle covering the coupling 22 can be included. Some possible thermal sensor locations are shown at T1, T2, T3, outflow, and inflow. It is expected that thermal sensors T1, T2, and T3 will be located within an ablation zone.

A challenge is to integrate the thermal sensors to the probe body 24 without increasing the overall diameter of the probe and/or creating a high back-pressure of coolant flowing inside the microwave ablation probe 20. Conventionally, very thin thermocouple wires would be used to implement such a design. There are multiple downsides when thermocouple wires are used in or on the probe body 24 such as high cost, difficulty in assembling the wires to make the thermocouple, the wires being fragile and hard to handle during assembly, difficultly in soldering to connectors or printed circuit boards, and separate wires needed for each thermal sensor.

By using a flexible circuit, such as that shown in FIG. 3, all the above-mentioned issues are mitigated. In addition, a more precise and repeatable placement of thermal sensors is achieved. For example, in a case where three thermal sensors are used in a conventional probe, there will be at least three wires if there is one for each thermal sensor. Each wire needs to be secured on the probe separately. During manufacturing, a technician needs to repeat the step of securing the thermal sensor wires on the probe three times. In a probe according to the present disclosure, the locations for three thermal sensors T1, T2, T3 can be one each at marker band 3, 4, and 5 cm, for example. In using a flexible circuit 30 there is only one assembly that includes the substrate SUB all three thermal sensors TC1, TC2, and TC3 (shown in FIG. 3). Because of the nature of a flexible circuit, all three thermal sensors TC1, TC2, TC3 are on the same substrate SUB at predefined geometric locations. Thus, the location of each and distance between adjacent thermal sensors TC1, TC2, TC3 is more precise than conventional assembly, as the precision of flexible circuit manufacturing is higher than a technician securing wires of thermal sensors. The accuracy of flexible circuit manufacturing can be within a tolerance of 2 mil. (0.002"). This means the traces on the flexible circuit 30 could be as small as 2 mil. or as close as 2 mil. to each other.

FIG. 3 shows a flexible circuit 30 implementation for the microwave ablation probe 20. The flexible circuit 30 can include a flexible substrate SUB on which metalized traces are routed to define an electrical circuit. The flexible substrate SUB can be polyimide or another suitable material. The metalized traces can include a top trace and a ground trace, for example, to create each thermal sensor TC1, TC2, TC3, Inflow TC, and Outflow TC.

As shown, the flexible circuit 30 can include five thermal sensors TC1, TC2, TC3 Inflow TC, and Outflow TC, although other numbers are possible. For example, in some embodiments, a thermal sensor can be located on cables/tubes routed between the microwave ablation probe 20 and an associated control console. In the embodiment shown, there are three legs in the flexible circuit 30. The longer leg contains three thermal sensors TC1, TC2, TC3 while the other two have only one each for Inflow TC and Outflow TC, respectively. If it is desired to add another thermal sensor, it is possible to add it to any of these legs or add a separate leg to the substrate SUB. The first option would only increase a leg width slightly. The thermal sensor on the new leg would be routed and attached to the desired location.

The traces defining the thermal sensors can be routed to terminal pads 32 for interface to a connector or other wiring leading to control electronics (not shown). Some common metals that can be used in the thermal sensors can be iron (Fe), copper (Cu), nickel (Ni), and platinum (Pt). Metal alloys, or combinations of metals, can also be used in thermal sensors. Nichrome is an alloy consisting of nickel and chromium (Cr). Constantan^{™} is a mixture of copper and nickel. Alumel^{™} is an alloy consisting of mostly nickel with small percentages of aluminum, manganese, and silicon.

An advantage of this implementation is integration of a connector and/or an array or row of terminal pads 32 into the flexible substrate SUB that replaces a conventional soldering step and facilitates connection to another connector or wiring. Another advantage is accurate and repeatable placement of the thermal sensors. Additionally, an analog to digital converter can be located in a handle or other suitable location to interface with the flexible circuit 30 to digitize the thermal sensor signals and reduce the wiring interface from the flexible circuit to the control console to two wires.

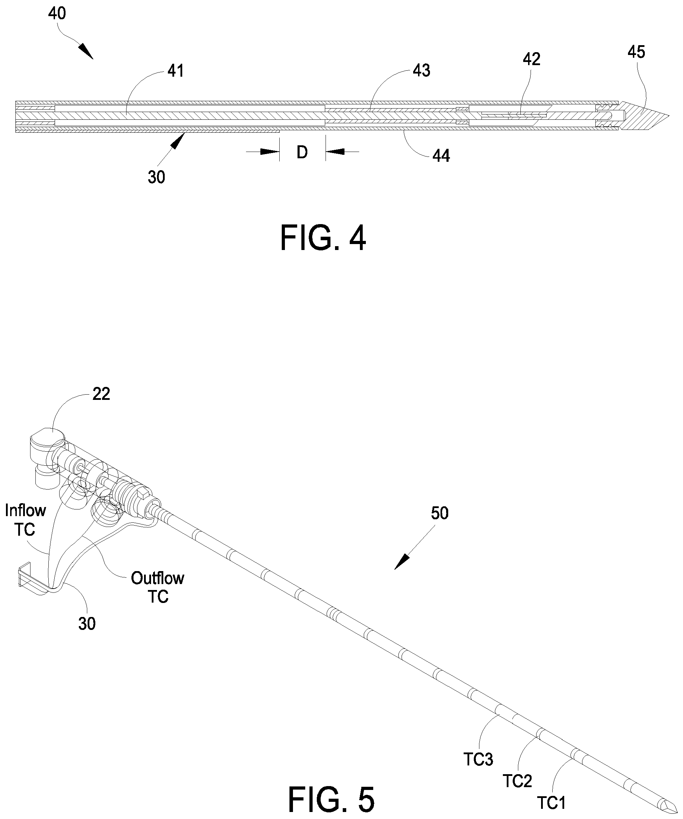

An example probe 40 is shown in FIG. 4. As shown, the probes 40 extends in a longitudinal direction along a central axis. The probes 40 includes a cable 41 positioned at a center along the central axis. The cable 41 is configured to deliver a current to an antenna 42 located toward a tip 45 that can deliver RF energy or microwaves to the target tissue during an ablation treatment. A choke 43 can be positioned before the antenna 42 along the cable 41 to limit and/or reduce the flow of current back along the cable 41. The probe 40 can also include a cooling tube that is positioned inside a shell 44 of the probe 40. The cooling tube can extend longitudinally over the choke 43 and the antenna 42. As shown, the flexible circuit 30 can be secured on the shell 44 by tacking with adhesive at a few points and then secured along the probe body 24 by using heat shrink tubing. Optionally, a pressure sensitive adhesive can be used. In this example, a distance D between a proximal end of the choke and the flexible circuit 30 should not be less than 3.6 mm. In designs without a choke, and end of the flexible circuit can be placed closer to the tip 45.

FIG. 5 is an example of a microwave ablation probe 50 that was built with a flexible circuit 30 including thermocouples as thermal sensors TC1, TC2, TC3, Inflow TC, and Outflow TC. The extended portion of the flexible circuit leading down the probe shaft to the thermal sensors TC1, TC2, and TC3 have been routed under the shell and are not visible. The thermal sensors TC1, TC2, and TC3 have been placed at marker band 3, 4, and 5 cm. on the probe shaft. To integrate the flexible circuit 30 to the probe shaft, TC1 is secured in place by an adhesive. Then, while keeping the flexible circuit 30 straight and untwisted, a heat shrink can be slid over the probe shaft. The thermal sensors Inflow TC and Outflow TC can be adhered to the coupling 22.

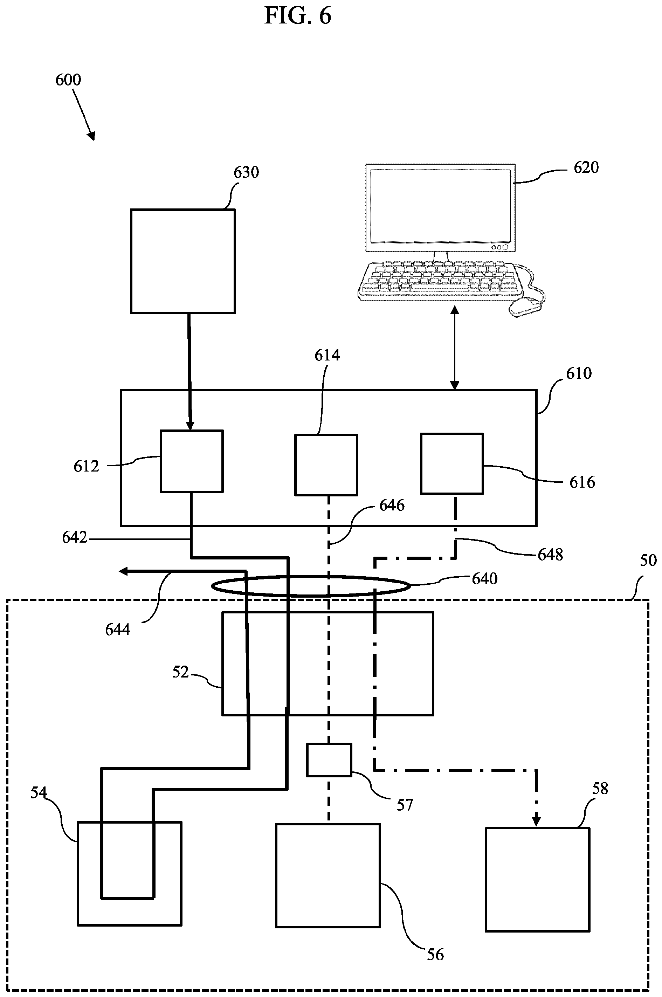

FIG. 6 is a functional block diagram of a microwave ablation system 600 according to an exemplary embodiment. As shown, the microwave ablation system 600 can include an ablation controller 610, an ablation computing system 620, a coolant reservoir 630, a flexible cable 640, and the microwave ablation probe 50. The ablation controller 610 can be coupled to the microwave ablation probe 50 via the flexible cable 640 to provide operation in a microwave mode. The ablation controller 610 can include a coolant delivery system 612, thermal sensor interface circuitry 614, and a microwave power generator 616.

The ablation computing system 620 can include a processor such as a computer, server, laptop, tablet, workstation, network, or the like. Additionally, the ablation computing system 620 and/or the ablation controller 610 can be in communication with a memory for storage of operating software, pre-treatment planning information, and data gathered during treatment. The memory can be a portion of the ablation computing system 620 and/or the ablation controller 610, a network, or a cloud. The ablation computing system 620 can also include a user interface such as display and/or other input-output devices to allow a user to interact, control, view, and otherwise configure the operation of the microwave ablation system 600. While the ablation controller 610 and the ablation computing system 620 are shown as separate elements in FIG. 6, it should be appreciated that these and other features can be combined into single structures or be further separated from that shown and described.

The coolant reservoir 630 can be defined as a container, tank, intravenous (IV) bag or any other structure suitable to hold a fluid capable of cooling the microwave ablation probe 50 and operate with the coolant delivery system 612. For example, the coolant can be distilled water, saline, or carbon dioxide (CO₂) gas.

The flexible cable 640 can include an inflow line 642 for coolant to flow from the coolant delivery system 612 to the microwave ablation probe 50, an outflow line 644 for coolant to flow from the microwave ablation probe 50 to the outside, sensor wiring 646 to transmit electrical signals between the thermal sensor interface 614 circuitry and the microwave ablation probe 50, and microwave wiring 648 to transmit microwave energy from the microwave power generator 616 to the microwave ablation probe 50. In an embodiment, the coolant reservoir 630 is an IV bag containing distilled water or saline where the inflow line 642 includes a spike for insertion into the IV bag, and the outflow line 644 returns to the IV bag. Thus, in this case, the cooling system is a closed circuit. In another embodiment, CO₂ gas is used as the coolant. In this case, the coolant reservoir 630 is a gas tank and the outflow line 644 and coolant gas does not return to the tank. Gas from the outflow line 644 is released to the environment in an open circuit.

The flexible cable 640 can include connectors suitable to connect to the ablation controller 610 and the microwave ablation probe 50.

The microwave ablation probe 50 can include a coupling 52, a coolant conduit 54, thermal sensor circuitry 56, an analog to digital converter 57, and a microwave antenna 58. The coupling 52 can include connection to the inflow line 642, the outflow line 644, the sensor wiring 646, and the microwave wiring 648. The cooling conduit 54 can be configured to route coolant through the microwave ablation probe 50 to absorb heat and cool the microwave ablation probe 50 during use. The thermal sensor circuitry 56 can be a flexible circuit including thermal sensors, as described herein, to communicate electrical signals representing temperature to the thermal sensor interface circuitry 614. In some embodiments, electrical signals from the thermal sensor circuitry 56 can be routed to the analog to digital converter 57. The analog to digital converter 57 can convert electrical signals from the thermal sensor circuitry 56 to a two-wire digital signal for transmission to the thermal sensor interface circuitry 614 to reduce the number of wires in the flexible cable 640 that would otherwise be required to transmit analog signals for multiple thermal sensors. The microwave antenna 58 can convert the microwave energy from the microwave power generator 616 to an electromagnetic field that radiates to heat tissue in the ablation zone.

The coolant delivery system 612 can be configured to draw or pump coolant from the coolant reservoir 630 to the microwave ablation probe 50 via the inflow line 642 of the flexible cable 640. Although shown as a portion of the ablation controller 610, the coolant delivery system 612 can be separated from the ablation controller 610.

The thermal sensor interface circuitry 614 can receive an electrical signal representing a temperature from the thermal sensor circuitry 56. This can be an analog signal or digital signal via the analog to digital converter 57. The ablation controller 610 can collect, analyze, store, and/or perform other operations with the temperature information from the thermal sensor circuitry 56.

The microwave power generator 616 is used to generate microwave signals that are transmitted to the microwave antenna 58. The microwave antenna 58 converts the fed high-power microwave currents into electromagnetic waves that radiate into its surrounding medium. In this manner, the microwave energy can be transferred to the target tissue and, in turn, heat the tissue at the distal end of the microwave ablation probe 50. The heat can be produced using various duty cycles and/or via various power profiles to elevate the temperature of the tissue to destroy the tissue or coagulate a bleeding condition.

The microwave ablation probe 50 was tested in the following cases: (i) no cooling fluid (i.e., saline) in the probe, (ii) probe priming, and (iii) occluded coolant flow. In each test, a microwave ablation probe 50 like that configured in FIG. 5 was connected to a microwave generator. A spike for the probe was inserted to an IV bag containing saline with the saline being set to return in a closed system and the probe was held in air. The microwave generator was turned on at 5 W power for a duration of 40 and 60 seconds with flow rates at 0, 50, and 100 mL/min and temperatures were measured with the thermocouples integrated to the microwave ablation probe 50.

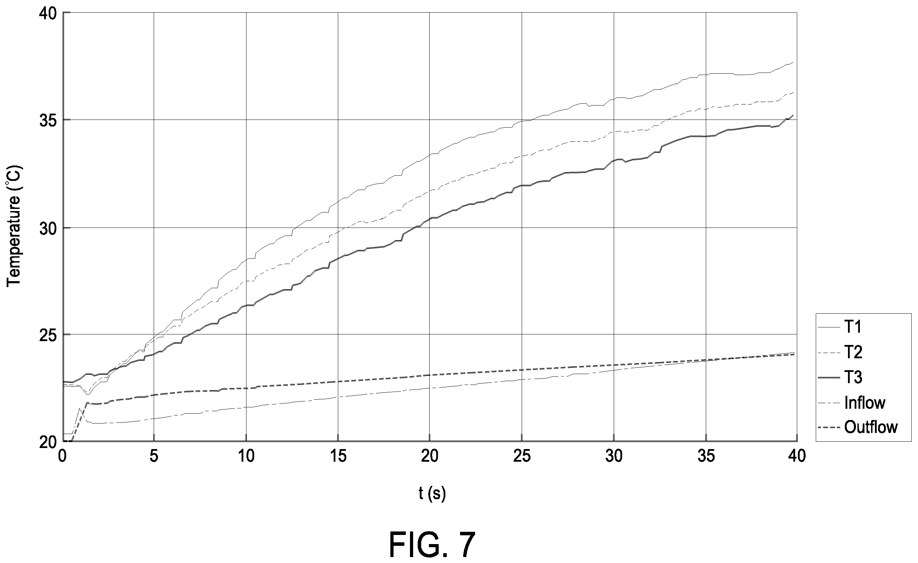

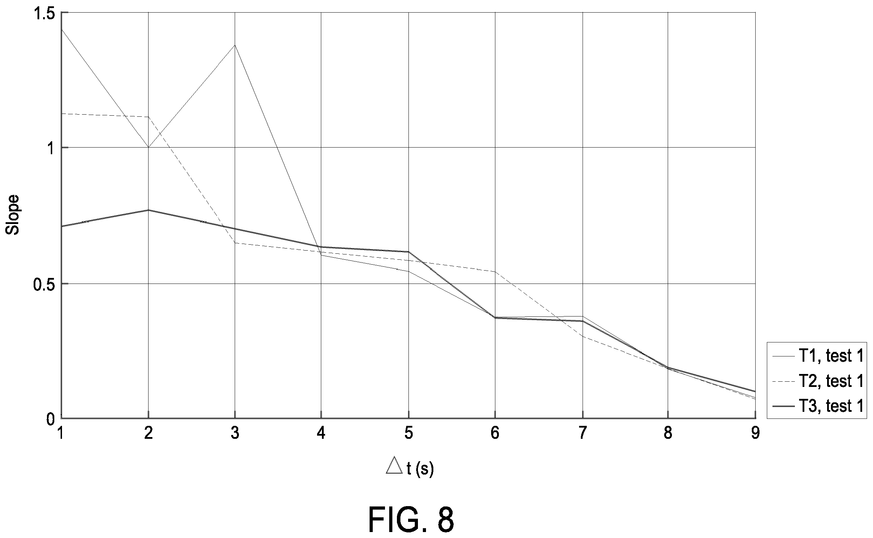

FIG. 7 is a graph of thermocouple temperature (°C) versus time (sec.) in the case with no saline present in the microwave ablation probe 50 at start up. As shown in FIG. 7, after 40 sec. a temperature rise was observed by thermocouples T1, T2, and T3 from about room temperature to about 35° C and above. The slope of temperature curves can be used to detect the presence of saline in the probe. As shown in FIG. 8, slopes calculated for T1, T2, and T3 for Δt = 4 sec. shows that a slope greater than 0.5 can be used to determine that no saline is present in the microwave ablation probe 50. The slope will be very close to zero in case of flow and presence of saline. No presence of saline could indicate a start-up issue. Because the cooling system is a closed circuit, the IV bag would be empty if there is leak. In case of a leak, the technician normally would detect the leak before the IV bag is completely depleted.

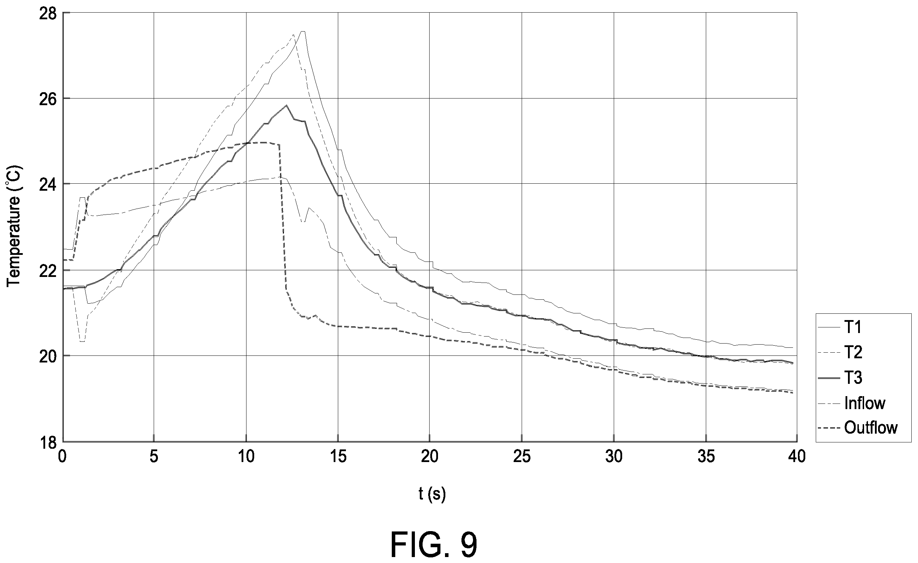

FIG. 9 is a graph of thermocouple temperature (°C) versus time (sec.) in the case to determine if the microwave ablation probe 50 was primed with saline. In this case, a coolant pump was turned on at the same time as the microwave generator. In the beginning of the test, a temperature rise was observed for the thermocouples. However, when saline reached the probe after about 12 sec., the thermocouple temperatures dropped, thus indicating that it takes about 12 sec. to prime the microwave ablation probe 50. By monitoring the slope of the temperatures for thermocouples T1, T2, and T3 and looking for a negative slope after a positive slope, presence of coolant flow in the microwave ablation probe 50 can be detected. In addition, a sharp drop in outflow temperature can be used to detect presence of coolant. As shown, the outflow trace in FIG. 9 has a sharp drop in temperature at about 12 sec. It is like a step change in the temperature right at the point when the slope of TC1 to TC3 traces becomes negative. It is noted that the temperature of the coolant reservoir and/or the flow rate of the coolant pump can be adjusted and used in controlling the microwave ablation probe temperature.

To emulate the case of occluded coolant flow, the microwave ablation probe 50 was filled with saline first and then the coolant pump was turn off. A temperature rise similar to FIG. 7 was measured by the thermocouples. Thus, it is possible to detect an occluded coolant flow situation with this thermocouple configuration.

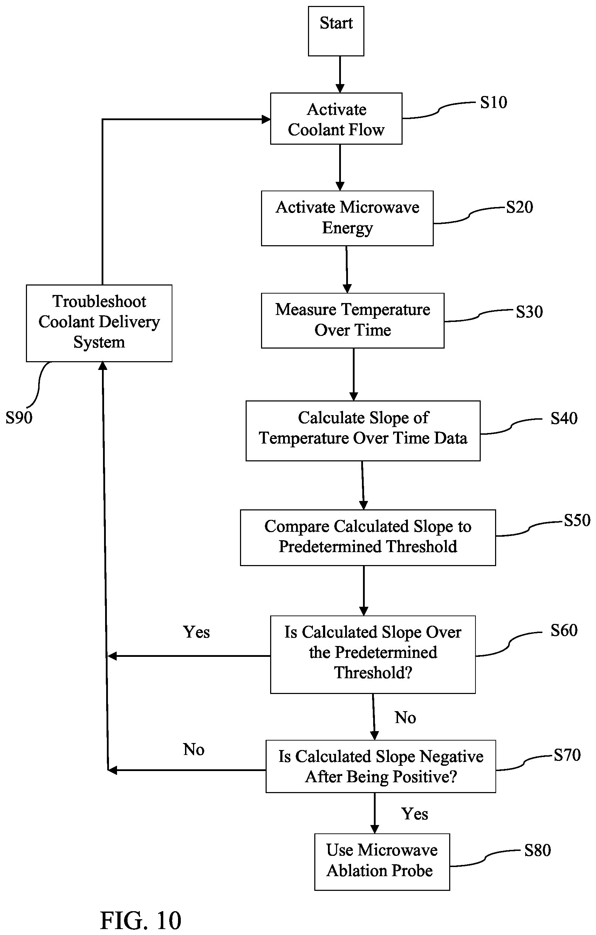

FIG. 10 is a flow chart of a method of monitoring a coolant status of a microwave ablation probe system according to an embodiment of the present disclosure, for example like the system 600 shown in FIG. 6 with a probe 50 like that shown in FIG. 5. In step S10, coolant flow can be activated to a microwave ablation probe. This can be accomplished by activating a coolant pump or any other action that is compatible with initiating the system to deliver the coolant to the microwave ablation probe. In step S20, microwave energy can be activated to be transmitted to the microwave ablation probe. This can be done by activating a microwave generator or by any other suitable action.

In step S30, temperature of thermal sensors located in the microwave ablation probe system can be measured and recorded over a period of time. For example, the period of time can be on the order of a few seconds up to 60 sec. For example, the period of time can be 1, 2, or 4 sec. The time and temperature data can be stored in a memory that is a portion of the microwave ablation probe system. In step S40, the time and temperature data can be operated by a computing system as a portion of the microwave ablation probe system to calculate a slope of the temperature over time for each of the thermal sensors. For example, the slopes can be calculated over a 4 sec time period. In step S50, the calculated slopes for each of the thermal sensors is compared to a predetermined threshold value. For example, the threshold value can be 0.5. In step S60, a determination is made if the calculated slopes are over the threshold. If the calculated slopes are at or above the threshold, an indication that the temperatures of the thermal sensors are rising too fast, the operator is instructed to troubleshoot the coolant delivery system at step S90 to ensure that the coolant reservoir contains coolant and that coolant is properly flowing. The computer system can show a message indicating insufficient cooling and instruct the user to check potential causes such as: if the spike of the probe is correctly inserted into the IV bag, if there is any leak, or if there is any occlusion on the coolant path. If the calculated slopes are not above the threshold, then, in step S70, a determination is made if the calculated slopes turn negative after being positive. If the calculated slopes are still positive, the operator is instructed to troubleshoot the coolant delivery system at step S90. Additionally, the computer system can be configured to not allow microwave to be turned on. User will not be able to use the probe until troubleshooting is completed. If the calculated slopes are negative after being positive, an indication that the coolant delivery system is working and providing cooling at the thermal sensor locations, at step S80, the microwave ablation probe is ready for use. As a result, the computing system is configured to monitor, process, or calculate the measured temperatures by the thermal sensors on the microwave ablation probe over time in order to determine if the coolant delivery system is cooling the ablation probe, and provide feedback on ablation procedure progress.

Many modifications and variations of the present invention are possible in light of the above teachings. It is therefore to be understood that within the scope of the appended claims the invention may be practiced otherwise than as specifically described.

## Claims

1. A microwave ablation probe system (600), comprising:
a microwave ablation probe (50),
a coolant delivery system (612) configured to deliver coolant to the microwave ablation probe (50);
a microwave generator (616) configured to deliver microwave energy to the microwave ablation probe (50); and
a computing system (620) in communication with a thermal sensor (T1, T2, T3) arranged on the microwave ablation probe (50) and configured to collect temperature data over time from the thermal sensor (T1, T2, T3), wherein
the computing system (620) is configured to calculate a slope of the temperature data over time to determine if the coolant delivery system (612) is cooling the microwave ablation probe (50), **characterized in that** the system further comprises a flexible circuit that includes the thermal sensor (T1, T2, T3),
wherein the flexible circuit (30) is attached to the microwave ablation probe (50).

2. The microwave ablation probe system of claim 1, wherein the flexible circuit (30) includes a plurality of thermal sensors (614).

3. The microwave ablation probe system of claim 1, wherein the coolant delivery system (612) includes a conduit through the microwave ablation probe (50).

4. The microwave ablation probe system of claim 3, wherein the microwave ablation probe (50) includes a coupling (52) to which the conduit is attached.

5. The microwave ablation probe system of claim 1, wherein the microwave ablation probe (50) includes:
an elongate shaft (24);
a coupling (22) attached to the elongate shaft (24) and the coolant delivery system (612); and
wherein the flexible circuit (30) including the thermal sensor (TC1, TC2, TC3) is attached to the elongate shaft (24).

6. The microwave ablation probe system of claim 5, wherein the flexible circuit (30) is attached to the coupling (22) and includes a further thermal sensor (Inflow TC, Outflow TC) at the coupling (22).

7. The microwave ablation probe system of claim 1, wherein the flexible circuit (30) is attached to the exterior surface of the microwave ablation probe (50).

8. The microwave ablation probe system of claim 5 or 6, further comprising a connector implemented on a same printed circuit board, PCB, as the thermal sensor (TC1, TC2, TC3) and/or the further thermal sensor (Inflow TC, Outflow TC).

9. The microwave ablation probe system of claim 8, wherein multiple thermal sensors are implemented on the same PCB.

## Patentansprüche

1. Mikrowellenablationssondensystem (600), umfassend:
eine Mikrowellenablationssonde (50);
ein Kühlmittelabgabesystem (612), das konfiguriert ist, um Kühlmittel an die Mikrowellenablationssonde (50) abzugeben;
einen Mikrowellengenerator (616), der konfiguriert ist, um Mikrowellenenergie an die Mikrowellenablationssonde (50) abzugeben; und
ein Rechensystem (620) in Kommunikation mit einem thermischen Sensor (T1, T2, T3), der an der Mikrowellenablationssonde (50) angeordnet und konfiguriert ist, um Temperaturdaten im Zeitverlauf von dem thermischen Sensor (T1, T2, T3) zu sammeln, wobei
das Rechensystem (620) konfiguriert ist, um eine Steigung der Temperaturdaten im Zeitverlauf zu berechnen, um zu bestimmen, ob das Kühlmittelabgabesystem (612) die Mikrowellenablationssonde (50) kühlt, **dadurch gekennzeichnet, dass** das System ferner eine flexible Schaltung umfasst, die den thermischen Sensor (T1, T2, T3) einschließt,
wobei die flexible Schaltung (30) an der Mikrowellenablationssonde (50) angebracht ist.

2. Mikrowellenablationssondensystem nach Anspruch 1, wobei die flexible Schaltung (30) eine Vielzahl von thermischen Sensoren (614) einschließt.

3. Mikrowellenablationssondensystem nach Anspruch 1, wobei das Kühlmittelabgabesystem (612) eine Leitung durch die Mikrowellenablationssonde (50) einschließt.

4. Mikrowellenablationssondensystem nach Anspruch 3, wobei die Mikrowellenablationssonde (50) eine Kupplung (52) einschließt, an der die Leitung angebracht ist.

5. Mikrowellenablationssondensystem nach Anspruch 1, wobei die Mikrowellenablationssonde (50) Folgendes einschließt:
einen länglichen Schaft (24);
eine Kupplung (22), die an dem länglichen Schaft (24) und dem Kühlmittelabgabesystem (612) angebracht ist; und
wobei die flexible Schaltung (30), die den thermischen Sensor (TC1, TC2, TC3) einschließt, an dem länglichen Schaft (24) angebracht ist.

6. Mikrowellenablationssondensystem nach Anspruch 5, wobei die flexible Schaltung (30) an der Kupplung (22) angebracht ist und einen weiteren thermischen Sensor (Inflow TC, Outflow TC) an der Kupplung (22) einschließt.

7. Mikrowellenablationssondensystem nach Anspruch 1, wobei die flexible Schaltung (30) an der Außenoberfläche der Mikrowellenablationssonde (50) angebracht ist.

8. Mikrowellenablationssondensystem nach Anspruch 5 oder 6, ferner umfassend einen Steckverbinder, der auf einer selben Leiterplatte (printed circuit board, PCB) wie der thermische Sensor (TC1, TC2, TC3) und/oder der weitere thermische Sensor (Inflow TC, Outflow TC) implementiert ist.

9. Mikrowellenablationssondensystem nach Anspruch 8, wobei mehrere thermische Sensoren auf derselben PCB implementiert sind.

## Revendications

1. Système de sonde d'ablation par micro-ondes (600), comprenant :
une sonde d'ablation par micro-ondes (50) ;
un système de distribution de fluide de refroidissement (612) configuré pour distribuer du fluide de refroidissement à la sonde d'ablation par micro-ondes (50) ;
un générateur de micro-ondes (616) configuré pour distribuer de l'énergie micro-ondes à la sonde d'ablation par micro-ondes (50) ; et
un système informatique (620) en communication avec un capteur thermique (T1, T2, T3) agencé sur la sonde d'ablation par micro-ondes (50) et configuré pour collecter des données de température au fil du temps à partir du capteur thermique (T1, T2, T3), dans lequel
le système informatique (620) est configuré pour calculer une pente des données de température au fil du temps afin de déterminer si le système de distribution de fluide de refroidissement (612) refroidit la sonde d'ablation par micro-ondes (50), **caractérisé en ce que** le système comprend en outre un circuit flexible qui comporte le capteur thermique (T1, T2, T3),
dans lequel le circuit flexible (30) est fixé à la sonde d'ablation par micro-ondes (50).

2. Système de sonde d'ablation par micro-ondes selon la revendication 1, dans lequel le circuit flexible (30) comporte une pluralité de capteurs thermiques (614).

3. Système de sonde d'ablation par micro-ondes selon la revendication 1, dans lequel le système de distribution de fluide de refroidissement (612) comporte un conduit traversant la sonde d'ablation par micro-ondes (50).

4. Système de sonde d'ablation par micro-ondes selon la revendication 3, dans lequel la sonde d'ablation par micro-ondes (50) comporte un couplage (52) auquel le conduit est fixé.

5. Système de sonde d'ablation par micro-ondes selon la revendication 1, dans lequel la sonde d'ablation par micro-ondes (50) comporte :
une tige allongée (24) ;
un couplage (22) fixé à l'arbre allongé (24) et au système de distribution de fluide de refroidissement (612) ; et
dans lequel le circuit flexible (30) comportant le capteur thermique (TC1, TC2, TC3) est fixé à l'arbre allongé (24).

6. Système de sonde d'ablation par micro-ondes selon la revendication 5, dans lequel le circuit flexible (30) est fixé au couplage (22) et comporte un capteur thermique supplémentaire (Inflow TC, Outflow TC) au niveau du couplage (22).

7. Système de sonde d'ablation par micro-ondes selon la revendication 1, dans lequel le circuit flexible (30) est fixé à la surface extérieure de la sonde d'ablation par micro-ondes (50).

8. Système de sonde d'ablation par micro-ondes selon la revendication 5 ou 6, comprenant en outre un connecteur mis en œuvre sur une même carte de circuit imprimé, PCB, que le capteur thermique (TC1, TC2, TC3) et/ou le capteur thermique supplémentaire (Inflow TC, Outflow TC).

9. Système de sonde d'ablation par micro-ondes selon la revendication 8, dans lequel de multiples capteurs thermiques sont mis en œuvre sur la même PCB.
